# EUROPÄISCHE PATENTSCHRIFT

(11) **EP 0 968 412 B1**
(45) Veröffentlichungstag und Bekanntmachung des Hinweises auf die Patenterteilung: **06.11.2002**
(21) Anmeldenummer: 98923995.9
(22) Anmeldetag: 11.03.1998
(51) Int. Cl.: G01N 21/64

(54) **VORRICHTUNG UND VERFAHREN ZUR DURCHFÜHRUNG VON FLUORESZENZIMMUNOTESTS**
DEVICE AND METHOD FOR CARRYING OUT FLUORESCENCE IMMUNOTESTS
DISPOSITIF ET METHODE POUR EFFECTUER DES ANALYSES IMMUNOLOGIQUES PAR FLUORESCENCE

(30) Priorität: 18.03.1997 DE 19711281
(43) Veröffentlichungstag der Anmeldung: 05.01.2000
(73) Patentinhaber: INSTITUT FUR CHEMO- UND BIOSENSORIK Münster E.V., 48149 Münster (DE)
(72) Erfinder: MEUSEL, Markus, D-48159 Münster (DE); TRAU, Dieter, D-48161 Münster (DE); KATERKAMP, Andreas, D-48149 Münster (DE)
(74) Vertreter: Pfenning, Meinig & Partner GbR
(86) Internationale Anmeldenummer: DE9800729
(87) Internationale Veröffentlichungsnummer: WO98041843

(56) Entgegenhaltungen:
- WO-A-90/05295
- WO-A-90/06503
- WO-A-94/27137
- DE-A- 3 814 370
- DE-C- 19 628 002

## Beschreibung

Die Erfindung betrifft eine Vorrichtung und ein Verfahren zur Durchführung von insbesondere quantitativen Fluoreszenzimmunotests mittels Evanescentfeldanregung. Dabei können verschiedenste bekannte biochemische Assays von allgemeinen Rezeptor-Ligand Systemen, wie Antikörper-Antigen, Lectin-Kohlenhydrat, DNA oder RNA-komplementäre Nukleinsäure, DNA oder RNA-Protein, Hormonrezeptor, Enzym-Enzymcofaktoren, Protein G oder Protein A-Immunglobin oder Avidin-Biotin zugrunde gelegt werden. Bevorzugt werden jedoch Antikörper-Antigen Systeme bewertet. Insbesondere können hochmolekulare und niedermolekulare Verbindungen (z.B. Haptene) nach der Erfindung detektiert werden.

Fluoreszenzimmunotests oder auch Fluoreszenzimmunosensoren werden üblicherweise bereits seit längerer Zeit eingesetzt und sie dienen dazu, hauptsächlich in einer flüssigen Probenmatrix eine unbekannte Menge einer bestimmten chemischen oder biochemischen Substanz zu quantifizieren. Dabei werden Antikörper selektiv an die zu bestimmende Substanz gebunden. Die zu bestimmende Substanz wird vom Fachmann auch als Antigen bezeichnet. Bei den Fluoreszenzimmunotests werden die analytspezifischen Antikörper mit einem Markierungsstoff markiert, der bei einer bestimmten stoffspezifischen Wellenlänge λₑₓ optisch angeregt wird und das Fluoreszenzlicht mit einer anderen Wellenlänge, die in der Regel größer ist, mit einem geeigneten Detektor unter Auswertung der Fluoreszenzlichtintensität genutzt. Die Ausnutzung der Evanescentfeldanregung bei der Durchführung solcher Fluoreszenzimmunotests respektive den Fluoreszenzimmunosensoren gehört bereits zum Stand der Technik. So sind verschiedene Lösungen bereits in WO 94/27137, von R.A. Badlay, R.A.L. Drake, I.A. Shanks, F.R.S., A.M. Smith und P.R. Stephenson in "Optical biosensors for immunoassays: fluorescence capillary-fill device", Phil. Trans. R. soc. Lund. B 316, 143 bis 160 (1987) und D. Christensen, S. Dyer, D. Fowers and J. Herron, "Analysis of Exitation and Collection Geometries for Planar Waveguide Immunosensors", Proc. SPIE-INt. Soc. Opt. Eng. Vol. 1936, Fiber Optic Sensors in Medical Diagnostics, 2 bis 8 (1993) beschrieben.

Des weiteren ist in WO 90/05295 A1 ein optisches Biosensorsystem beschrieben. Bei diesem System wird eine oder es werden mehrere Proben, unter Verwendung von Pumpen und Ventilen durch Kanäle zu einer oder mehreren Durchflußmeßzellen geführt. Dabei sind diese Durchflußmeßzellen nach oben offen und durch einen darüber angeordneten optischen Aufbau können Biomoleküle quantitativ detektiert werden. Für die Messung nachfolgender neuer Proben, macht sich demzufolge ein erheblicher Reinigungsaufwand erforderlich, um entsprechende Meßfehler zu vermeiden. Eine gegebenenfalls erforderliche Vorbereitung einer solchen Probe muß generell außerhalb dieses Systems vor der eigentlichen Messung durchgeführt werden, da keine hierfür geeigneten Elemente oder Maßnahmen genannt sind.

In WO 90/06503 ist ein Sensor beschrieben, bei dem Anregungslicht in einem geeigneten Winkel durch ein optisch transparentes Substrat auf eine Grenzfläche zu einer optisch transparenten Pufferschicht gerichtet wird, über der eine zusätzliche Wellenleiterschicht aufgebracht ist, an der wiederum die zu bestimmenden Analyten gebunden werden.

Der Brechungsindex der Pufferschicht ist dabei kleiner, als der des Substrates und des Wellenleiters. An der Grenzschicht Substrat/Puffer erfolgt Totalreflexion durch geeignete Wahl des Winkels des Anregungslichtes und über das dabei entstehende evanescente Feld wird das Anregungslicht in den über der Pufferschicht befindlichen Wellenleiter eingekoppelt. Das eingekoppelte Licht in den Wellenleiter wird über Totalreflexion im Wellenleiter geführt und das dabei sich ausbildende evanescente Feld wird entsprechend zur Fluoreszenzanregung genutzt.

Die Probe kann in einem oder mehreren Hohlräumen aufgenommen sein, wobei die entsprechende Dimensionierung eines solchen Hohlraumes nur dahingehend eingeschränkt wird, daß seine Größe den Probentransport in den Hohlräumen mittels Kapillarkraft ermöglicht. Nach der Probenaufnahme in den Hohlräumen erfolgt kein weiterer Fluß oder Bewegung der Probe.

Die bekannten Lösungen haben jedoch generell den Nachteil, daß sie nur für bestimmte Assayformate geeignet und ein aufwendiger Aufbau mit entsprechender Verfahrensführung erforderlich ist.

Es ist daher Aufgabe der Erfindung, eine Möglichkeit zu schaffen, mit der sehr einfach aufgebauten Vorrichtung quantitative Fluoreszenzimmunotests mit verschiedenen biochemischen Assays durchführen zu können.

Erfindungsgemäß wird diese Aufgabe durch die im Anspruch 1 enthaltenden Merkmale für die Vorrichtung und den Merkmalen des Anspruchs 11 für das Verfahren gelöst. Vorteilhafte Ausgestaltungsformen und Weiterbildungen der Erfindung ergeben sich mit der Verwendung der in den untergeordneten Ansprüchen enthaltenen Merkmale.

Bei einer in der DE 196 11 025 beschriebenen Vorrichtung wird Licht mindestens einer Lichtquelle unter einem Winkel α auf die Grenz fläche zweier Medien mit unterschiedlichem Brechungsindex gerichtet. Dabei wird eine Lichtquelle ausgewählt, die nahezu monochromatisches Licht mit einer Wellenlänge aussendet, die geeignet ist, den Markierungsstoff, in diesem Fall das Fluorophor, anzuregen. Als Lichtquelle eignen sich dabei insbesondere Laserdioden, da diese ein geeignetes Strahlprofil und eine ausreichende Lichtleistung, bei kleiner Baugröße und geringem Energieverbrauch aufweisen.

Es können aber auch andere monochromatisches Licht aussendende Lichtquellen Verwendung finden.

Der Winkel α mit dem das ausgesendete Licht auf die Grenzfläche gesendet wird, bestimmt neben dem Brechungsindex des im Strahlengang vor der Grenzfläche angeordneten Materials und dem sich daran anschließenden Material gemeinsam mit der Wellenlänge des Lichtes, die Eindringtiefe d für das evanescente Feld. Dabei muß der Brechungsindex n₁ des Materials, das im Strahlengang vor der Grenzfläche angeordnet ist Totalreflexion an der Grenzfläche ermöglichen und sollte daher größer als der Brechungsindex n₂ des anderen, danach angeordneten Materials sein. Der Winkel α wird bevorzugt so gewählt, daß gilt: sin(α) > n₂/n₁. Wird diese Voraussetzung erfüllt, wird alles Licht an der Grenzfläche reflektiert und somit Totalreflexion erreicht. Bei Erfüllung dieser Bedingung, dringt jedoch ein relativ kleiner Teil des Lichts durch die Grenzfläche in das Material, das im Strahlengang nach der Grenzfläche angeordnet ist, ein und das evanescente Feld entsteht. Durch das evanescente Feld werden nur solche Markierungsstoffe optisch angeregt, die sich in unmittelbarer Nähe der Grenzfläche befinden. Für die Durchführung der Fluoreszenzimmunotests ergibt sich daraus, daß ausschließlich die Markierungsstoffe der Antikörper oder Antigene angeregt werden, die auf der Oberfläche der Grenzfläche gebunden sind. Die Fluoreszenzintensität des von diesen Fluorophoren emittierten Lichtes ist damit direkt proportional zur Konzentration der an der Oberfläche gebundenen markierten Antikörper oder Antigene und je nach dem benutzten biochemischen Assay proportional oder umgekehrt proportional zur Antigenkonzentration.

Die in DE 196 11 025 beschriebene Vorrichtung verwendet nunmehr mindestens eine Lichtquelle, die nahezu monochromatisches Licht aussendet und dieses in einem, die Eindringtiefe d für das evanescente Feld vorgebenden Winkel α auf eine für dieses Licht transparente Bodenplatte richtet. Der Brechungsindex n₁ der Bodenplatte sollte größer als 1,33 sein. Auf der anderen Seite der Bodenplatte wird zwischen einer Deckplatte ein küvettenförmig ausgebildeter Aufnahmebereich ausgebildet. Zwischen Bodenplatte und dem küvettenförmig ausgebildeten Aufnahmebereich ist besagte Grenzfläche ausgebildet und das evanescente Feld kann sich mit der vorgegebenen Eindringtiefe d innerhalb des küvettenförmigen Aufnahmebereiches auf an die Oberfläche gebundenen markierten chemischen oder biochemischen Partner eines allgemeinen Rezeptor-Ligand Systems auswirken und die als Markierungsstoff verwendeten Fluorophore anregen.

Die so hervorgerufene Fluoreszenz wird mit der entsprechenden Intensität mit einem Detektor gemessen. Der Detektor ist dabei an der gleichen Seite der Bodenplatte wie die Lichtquelle angeordnet.

Als Detektor kann dabei ein einzelner lichtempfindlicher Detektor, eine lineare oder eine flächenhafte Anordnung von mehreren lichtempfindlichen Detektoren verwendet werden.

Dort wird ebenfalls beschrieben, daß es vorteilhaft ist, polarisiertes Licht auf die zu bestimmende Probe zu richten. Hierfür kann in den Strahlengang des Lichtes im Anschluß an die Lichtquelle ein Polarisator angeordnet werden.

Der Abstandshalter und ggf. zu verwendende Trennschichten weisen eine Dicke von 0,001 bis 10 mm, besonders bevorzugt 50 µm auf und eine Aussparung im Abstandshalter bildet den Aufnahmebereich für die Probe aus. Abstandshalter und Trennschichten können bevorzugt ein biokompatibler Klebefilm sein, der beidseitig haftend ausgebildet ist.

Das erfindungsgemäße Verfahren beruht im wesentlichen darauf, daß ein definiertes Probenvolumen durch den küvettenförmigen Aufnahmebereich geführt und dort einer Evanescentfeldanregung unterzogen wird, wie dies bereits beschrieben wurde. Das Probenvolumen kann dabei durch Saug-, Druckwirkung oder Kappilarkräfte durch den küvettenförmigen Aufnahmebereich und die funktionelle(n) Schicht(en) geführt werden.

In einer vorteilhaften Ausführung ist in einer Deckplatte zumindest eine Zuflußöffnung vorgesehen, in die ein Probencontainer einsetzbar oder angeordnet sein kann, dabei ist die Öffnung in der Deckplatte so angeordnet, daß eine Verbindung zwischen Zuflußöffnung bzw. Probencontainer und Aufnahmebereich herstellbar ist. Zusätzlich ist eine zweite Öffnung vorhanden, die ebenfalls mit dem küvettenförmigen Aufnahmebereich verbunden ist und einen Abfluß darstellt.

Die zweite Öffnung kann ebenfalls in der Deckplatte vorgesehen sein. An diese zweite Öffnung kann eine externe Pumpe angeschlossen oder eine eigene Pumpe eingesetzt werden.

Die Erfindung zeichnet sich dadurch aus, daß ein relativ einfach aufgebautes Grundmuster einer erfindungsgemäßen Vorrichtung in verschiedenster Form variiert bzw. verwendet werden kann. So können die wesentlichen Elemente Boden-, Deckplatte und Abstandshalter mit küvettenförmigen Aufnahmebereich durch funktionelle Schichten, die gegebenenfalls durch zwischen liegend angeordnete Trennschichten, jedoch einen Probendurchfluß erlaubend, in verschiedenster Form kombiniert werden. Eine oder mehrere solcher funktioneller Schichten können aber auch im Zuflußbereich für die Probe zum Aufnahmebereich angeordnet sein, wobei zu diesem Zuflußbereich eine Zuflußöffnung oder eine Verbindung zwischen einem in die Zuflußöffnung einsetzbaren Probencontainers bzw. die Verbindung von Zuflußöffnung und Aufnahmebereich, gehört.

Mit der Erfindung können die verschiedenen Assay-Formate durchgeführt und dabei hoch- und niedermolekulare Verbindungen gleichermaßen detektiert werden. Es können alle bekannten Assay-Formate, wie Sandwich-Titrations-/Kompetitions- und Verdrängungsformat durchgeführt werden.

Für den Fall, daß Trennschichten zwischen funktionellen Schichten bzw. diese einschließende Trennschichten verwendet, müssen diese entsprechend korrespondierende Durchbrechungen aufweisen, so daß gesichert ist, daß das Probenvolumen die gesamte Vorrichtung durchströmen kann. Als Trennschichten können beispielsweise Klebefolien verwendet werden, in die Durchbrechungen durch Ausstanzen hergestellt werden.

Nachfolgend soll die Erfindung beispielhaft näher beschrieben werden.

Dabei zeigt:
- Fig. 1: einen Teil einer Vorrichtung zur Aufnahme einer Probe, auf der die Erfindung aufgebaut;
- Fig. 2: eine schematische Darstellung einer Ausführungsform einer Vorrichtung nach Figur 1 mit zwei Lichtquellen;
- Fig. 3: eine Vorrichtung nach Figur 1 mit Probencontainer;
- Fig. 4: eine Vorrichtung nach Figuren 1 und 3 mit zylinderförmigen Hohlkörper;
- Fig. 5: eine erfindungsgemäße Vorrichtung mit zusätzlichen funktionellen Schichten mit lateralem Durchfluß;
- Fig. 6: eine erfindungsgemäße Vorrichtung mit mehreren funktionellen Schichten und transversalem Durchfluß;
- Fig. 7+8: zeitabhängige Fluoreszenzintensitätsverläufe;
- Fig. 9: ein Sandwich-Assayformat;
- Fig. 10: ein weiteres Sandwich-Assayformat;
- Fig. 11: ein Tritations- bzw. Kompetitions Assayformat;
- Fig. 12 + 13: ein Kompetitions- bzw. Verdrängungs-Assayformat mit direkt proportionalem Verhältnis von Analytkonzentration und Signalintensität;
- Fig. 14: ein Assayformat unter Nutzung einer zusätzlichen festen Phase;
- Fig. 15: einen Verdrängungsassay mit zusätzlicher fester Phase;
- Fig. 16: einen weiteren Verdrängungsassay und
- Fig. 17: eine allgemeine Legende für die in der Figur 9 bis 16 gezeigten Assayformate.

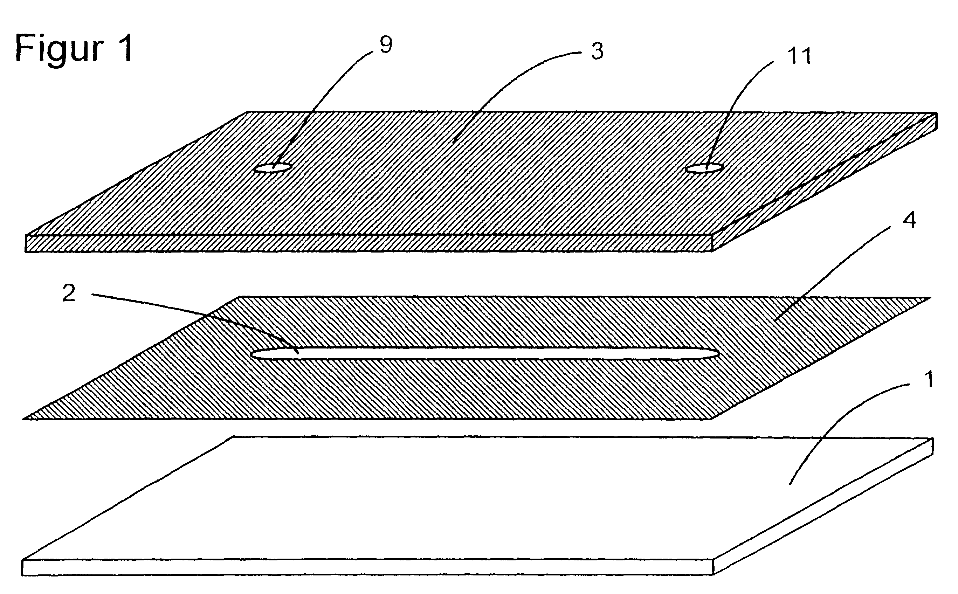

In der Figur 1 ist der prinzipielle Aufbau eines Teiles einer Vorrichtung, auf der die Erfindung aufbaut, dargestellt. Die dort abgebildeten drei Teile, die Bodenplatte 1, der Abstandshalter 4 und die Deckplatte 3, können vor der Durchführung des Fluoreszenzimmunotestes miteinander verbunden werden oder bilden bereits eine fertig komplettierte Einheit und gleichen in ihrem Aufbau einer Durchflußzelle und einer Meßküvette.

Dabei besteht die Bodenplatte 1 aus einem hochbrechenden transparenten Material, wie beispielsweise Glas oder einem Kunststoff, wie einem Polymer (PMMA oder PC) mit einem Brechungsindex n₁ > 1,33. Die Dicke der Bodenplatte kann in einem Bereich von 0,01 bis 10 mm, bevorzugt zwischen 0,5 und 1 mm liegen.

Der Abstandshalter 4 ist bevorzugt eine dünne Folie, die beidseitig mit einem Klebefilm versehen ist, oder ein dünner Klebefilm ist einmal auf die Bodenplatte 1 und zum anderen auf die Deckplatte 3 aufbringbar. Die gesamte Dicke des Abstandshalters inklusive des verwendeten Klebemittels sollte in einem Bereich zwischen 0,001 bis 10 mm, bevorzugt zwischen 0,01 und 0,2 mm und ganz besonders bevorzugt bei einer Dicke von 50 µm liegen. In den Abstandshalter 4 ist eine Durchbrechung herausgearbeitet, die einen küvettenförmigen Aufnahmebereich 2 ausbildet.

In der Figur 1 ist außerdem die Deckplatte 3 in der durchgehende Öffnungen 9 und 11, bei diesem Beispiel als Bohrungen ausgebildet, erkennbar. Auf deren Funktion wird später noch zurückzukommen sein. Die Öffnungen 9 und 11 sind dabei so angeordnet, daß sie zumindest teilweise den Bereich des Aufnahmebereiches 2 des Abstandshalters 4 übergreifen. Der Abstandshalter 4 kann vorzugsweise auch aus einem biokompatiblen Klebefilm, der bevorzugt beidseitig mit einer abziehbaren Schutzschicht versehen ist und bereits kommerziell erhaltbar ist, bestehen.

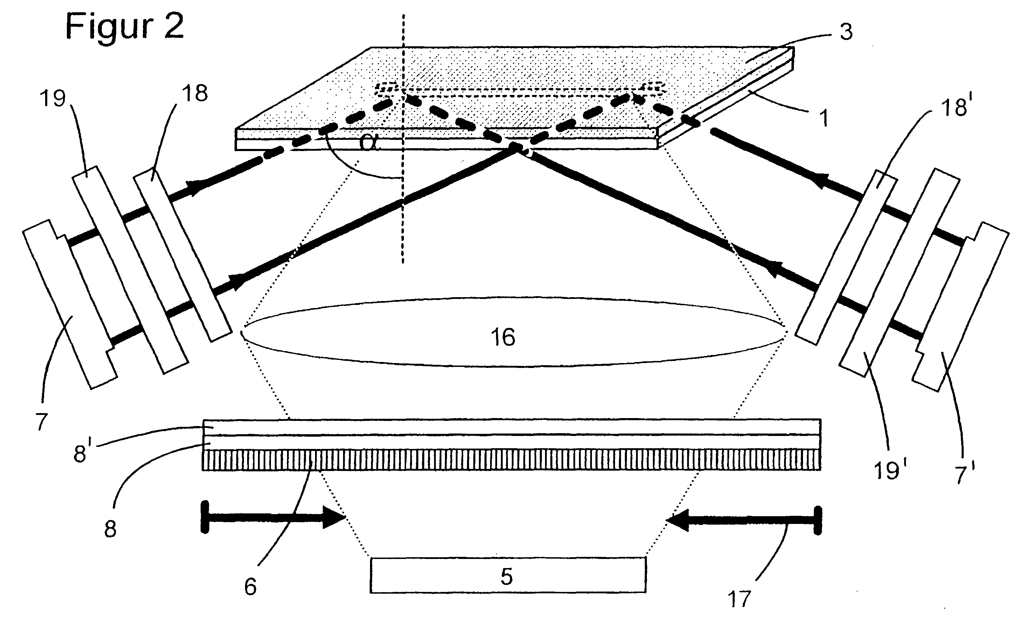

In der Figur 2 ist die Vorrichtung nach Figur 1 mit zwei Lichtquellen 7, 7', Filter 19, 19' und Polarisatoren 18, 18'dargestellt . Die Lichtquelle 7' sendet Licht einer Wellenlänge, die sich von der ersten Lichtquelle 7 unterscheidet. Bei diesem Beispiel wird vorzugsweise polarisiertes Licht verwendet. Die in der Figur 2 gezeigte Vorrichtung kann vorteilhaft eingesetzt werden, wenn unterschiedliche Markierungsstoffe, die bei verschiedenen Wellenlängen angeregt werden können, verwendet werden. Beispiele hierfür sind die Fluorophore Cy5 und Cy7. Dabei wird zur Anregung des Fluorophor Cy5 eine Laserdiode mit einem Licht einer Wellenlänge zwischen 635 und 655 nm und für die Fluorophore Cy7 eine Laserdiode, die Licht mit einer Wellenlänge zwischen 730 bis 780 nm sendet, verwendet.

Die Messung erfolgt bei dieser Ausführungsform in der Weise, daß die Dioden 7, 7' entweder alternierend geschaltet oder beispielsweise entsprechend synchronisierte Chopper eingesetzt werden, so daß gesichert ist, daß jeweils nur Licht einer Lichtquelle 7 oder 7' zur Anregung auf die Probe gelangen kann und dadurch keine Verfälschungen auftreten.

Da aber hierbei zwei verschiedene Fluoreszenzsignale das gleiche Filter passieren müssen, kann ein breitbandiges Filter 8 nicht mehr verwendet werden. Es sollten daher zwei Filter 8, 8' nacheinander angeordnet werden, die selektiv die Wellenlängen der anregenden Lichtquellen 7, 7' sperren. Hierfür können z.B. Notch-Filter verwendet werden.

Mit dieser Anordnung kann einmal ein Referenzsignal erhalten werden, das eine interne Kalibrierung des Meßsignals ermöglicht. Zur Referenzmessung wird ein Referenzantikörper, der nicht gegen ein Antigen aus der Probe gerichtet ist, verwendet. Der Referenzantikörper ist vorab quantifiziert und mit einem unterschiedlichen Markierungsstoff vom zu bestimmenden analytspezifischen Antikörper Ak unterscheidbar gemacht. Die Menge der an die Oberfläche tatsächlich gebundenen Referenzantikörper kann mit einer zweiten Lichtquelle 7', die Licht einer Fluoreszenz des unterschiedlichen Markierungsstoffes hervorruft, einem zweiten Streulichtfilter 8' und dem Detektor 5 bestimmt werden. Mit dieser Bestimmung können die Verluste der nicht an die Oberfläche gebundenen markierten analytspezifischen Antikörper Ak bzw. Antigene Ag berücksichtigt werden.

Neben der Gewinnung eines Referenzsignales können aber auch zwei unabhängig voneinander laufende Immunotests durchgeführt werden, wobei die Unterscheidung an Hand der unterschiedlichen Fluorophore erfolgt.

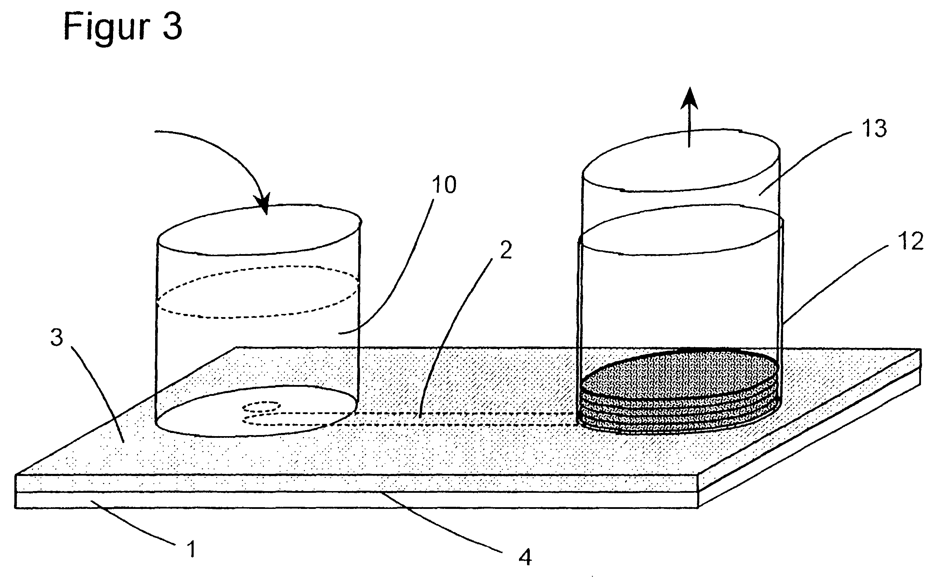

In der Figur 3 ist dargestellt, wie ein Probencontainer 10 zur Öffnung 9 in der Deckplatte 3 einer Vorrichtung nach Figur 1 angeordnet ist und so eine Verbindung zwischen Probencontainer 10 über Öffnung 9 zum Aufnahmebereich 2 herstellbar ist. Dabei bildet der Probencontainer 10 den Behälter, in dem die bekannte Menge mit dem Markierungsstoff Fluorophor markierte Biokomponente in der zu bestimmenden Probe vermischt werden. Dabei kann der Probencontainer 10 das Probenvolumen eindeutig definieren und so mit einem festen und bekannten Probenvolumen eine quantitative Aussage über die Antigenkonzentration erhalten werden kann. Der Probencontainer 10 muß daher immer mit der gleichen Menge befüllt werden, um reproduzierbare Ergebnisse erhalten zu können. Günstig sollte er dabei immer maximal befüllt werden. Bei einigen durchführbaren Assayformaten befindet sich die spezifische Biokomponente jeweils auf der Oberfläche des Probencontainers 10 und durch den Kontakt mit der flüssigen Probe löst sich diese von der Oberfläche und gelangt in die Probe. Weiterhin können sich die Biokomponenten auf zusätzlichen festen Phasen im Probencontainer 10 befinden. Eine einfache und bereits bekannte Methode besteht darin, lyophilisierte Antikörper auf die Oberfläche des Probencontainers 10 aufzubringen. Dadurch wird es möglich, das Ganze relativ lange vor der eigentlichen Durchführung des Immunotests zu lagern. Der Aufnahmebereich 2 definiert die Oberfläche auf der Bodenplatte 1, auf der je nach Assayformat die jeweils entsprechenden chemischen oder biochemischen Substanzen immobilisiert werden.

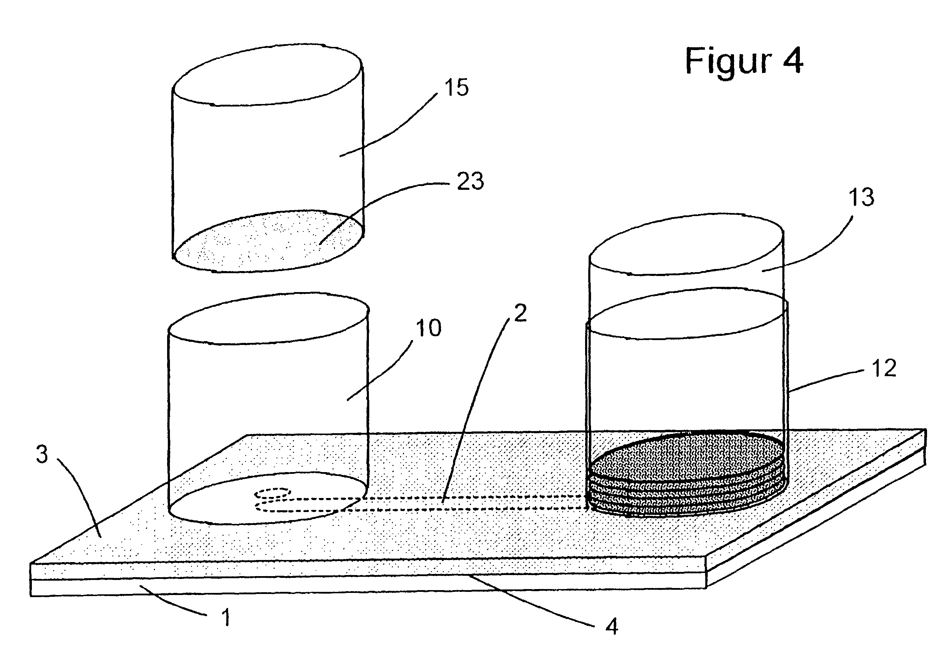

In der Figur 4 ist ebenfalls ein bevorzugt zylinderförmiger Hohlkörper 12, in dem ein Kolben 13 oder eine andere geeignete Abdeckung aufgenommen ist, dargestellt, die beide zusammen als Pumpe dienen. Wird der Kolben 13 aus dem zylinderförmigen Hohlkörper 12 herausbewegt, entsteht ein Unterdruck, der Probenmaterial aus dem Probencontainer 10 durch den Aufnahmebereich 2 in Richtung auf den zylinderförmigen Hohlkörper 12 saugt. Durch Kapillarkräfte im Aufnahmebereich 2 und durch ein saugfähiges Vlies wird der Fluß aufrechterhalten, bis das gesamte Probenvolumen durch den Aufnahmebereich 2 gefördert worden ist. Der zylinderförmige Hohlkörper 12 ist aufgesetzt oder hat ein Loch im Boden, so daß eine Verbindung zum Aufnahmebereich 2 vorhanden ist. Dies kann durch die zweite Öffnung 11 als Anschlußmöglichkeit in der Deckplatte 3 realisiert werden. Wird keine Deckplatte 3 verwendet, kann die Anschlußmöglichkeit auch anders ausgebildet sein.

Es kann aber auch an die Öffnung 11 eine externe andere Pumpe angeschlossen werden.

Nach Aufbringen der Probe (mit dem Probencontainer 10) muß eine entsprechende Zeit gewartet werden, so daß die gewünschte Bindung zwischen den Partnern eines allgemeinen Rezeptor-Ligand Systems vollständig erfolgen kann. Im Anschluß daran wird die Pumpe 12, 13 aktiviert und es wird gewartet, bis die gesamte Flüssigkeit durch den Aufnahmebereich 2 gepumpt worden ist. Nach Anregung mit der Lichtquelle 7 bzw. den Lichtquellen 7 und 7' kann dann die Antigenkonzentration bestimmt werden, wobei der erfindungsgemäße Aufbau, wie er in der Figur 2 dargestellt worden ist, angewendet werden soll.

Der Aufbau, wie er bisher dargestellt und beschrieben worden ist, kann für die verschiedensten biochemischen Assay's genutzt werden, wobei auf weitere Beispiele noch zurück zukommen sein wird.

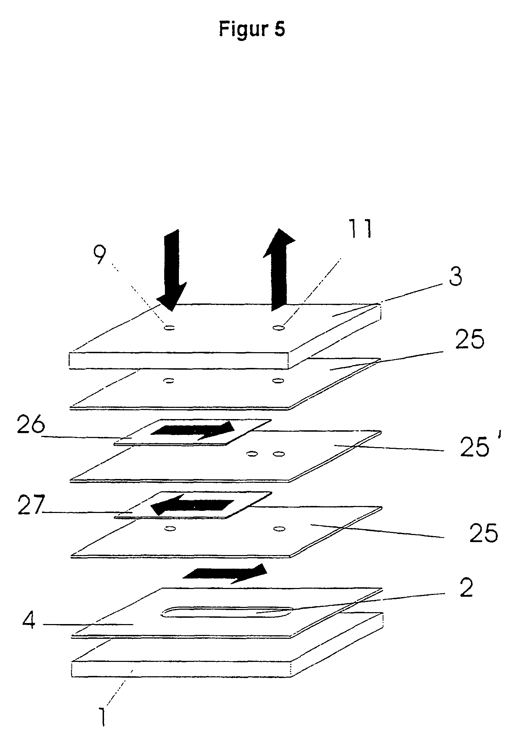

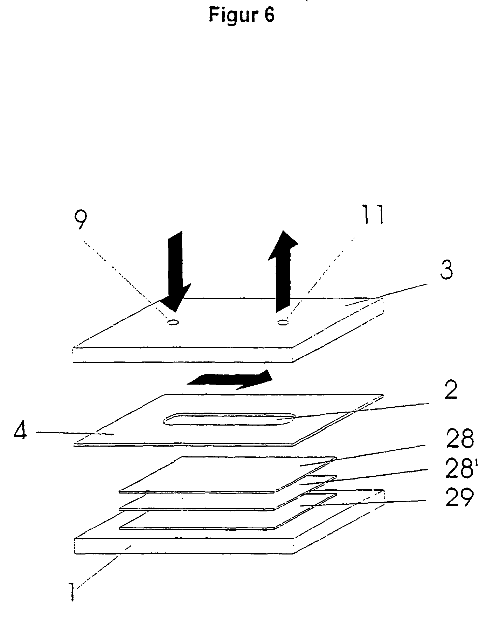

Wie dies insbesondere den Figuren 5 und 6 zu entnehmen ist, kann der wesentliche Teil der erfindungsgemäßen Vorrichtung sehr variabel ausgestaltet werden. So können die verschiedenen Elemente (Platten, Schichten) aus einem Bausatz in den verschiedensten Konfigurationen zusammengesetzt und dementsprechend für verschiedene Assayformate nach Bedarf vor Ort zur Verfügung gestellt werden.

So zeigt Figur 5 ein Beispiel einer erfindungsgemäßen Vorrichtung, bei dem zusätzliche funktionelle Schichten mit Lateraldurchfluß dargestellt ist. Dabei sind zusätzlich funktionelle Schichten 26 und 27 und trennende Schichten 25, 25' in den, bereits bei der Beschreibung der Figur 1 erklärten Aufbau einbezogen. Bei diesem Beispiel wurden zwei funktionelle Schichten 26 und 27 übereinander angeordnet, wobei sie allseitig von Trennschichten 25 und 25' umschlossen sind. Die Trennschichten 25 und 25' können dabei bevorzugt, wie bereits am anderen Ort beschrieben, als Klebefolien ausgebildet sein, in die Durchbrechungen ausgebildet sind. Diese Durchbrechungen dienen dazu, daß eine Verbindung zwischen Zuflußöffnung 9, den funktionellen Schichten 26, 27, dem Aufnahmebereich 2 und der Abflußöffnung 11 möglich wird. Die Durchflußrichtung geben dabei die in der Figur 5 eingezeichneten Pfeile wieder.

Eine Anpassung an verschiedene Assayformate kann durch Variation der Anordnung und/oder Auswahl der funktionellen Schichten 26, 27 erreicht werden. So können die funktionellen Schichten 26 und 27 beispielsweise Reagenzienreservoir bzw. eine reine Reaktionsschicht sein.

Es besteht aber auch die nicht dargestellte Möglichkeit, mindestens zwei unterschiedliche funktionelle Schichten in einer Ebene anzuordnen, so daß sie nacheinander durchflossen werden.

Der in der Figur 6 gezeigte Aufbau eines Teiles einer erfindungsgemäßen Vorrichtung unterscheidet sich von dem in der Figur 5 gezeigten Beispiel dadurch, daß ein transversaler Durchfluß erreicht werden kann. Bei diesem Beispiel sind drei funktionelle Schichten 28, 28' und 29 unmittelbar übereinander, d.h. ohne Trennschichten, direkt auf der Bodenplatte 1 angeordnet. Innerhalb des so ausgebildeten Schichtstapels aus funktionellen Schichten 28, 28' und 29 ist bei diesem Beispiel der Abstandshalter 4 mit dem küvettenförmigen Aufnahmebereich 2 unterhalb der Deckplatte 3 angeordnet. Auch hier geben die in der Figur 6 eingezeichneten Pfeile die Durchflußrichtung an.

Abweichend von dem in der Figur 6 dargestellten Aufbau, können selbstverständlich auch andere Anordnungen, die einen transversalen Durchfluß sichern, aufgebaut werden. Dabei können die funktionellen Schichten, wie bereits erwähnt, in ihrer Anzahl, Anordnung und Funktionsauswahl variiert werden. Die Anordnung kann entgegengesetzt zum gezeigten Beispiel auch oberhalb des Abstandshalters 4 ausgeführt werden.

Auch bei diesem Beispiel können Trennschichten eingesetzt werden, wobei jedoch zu beachten ist, daß ein transversaler Durchfluß nicht behindert werden darf. Die funktionellen Schichten können wiederum als Reagenzienreservoir bzw. Reaktionsschicht dienen.

Die erfindungsgemäß zu verwendenden funktionellen Schichten haben dabei den Vorteil, daß ein komplettes integriertes Meßsystem entsteht und lediglich die Probe durch den Aufbau geleitet werden muß.

Auch Kombinationen von funktionellen Schichten mit transversalem oder lateralem Durchfluß (Kombination der Beispiele Figur 5 und 6) sind möglich.

Die funktionellen Schichten 26, 27, 28, 28' und 29 können die Aufgaben der Probenvorbereitung (Puffern, Filtration, Separation, Eliminierung von Interferenzen u.a.), als Reagenzträgerschicht (z.B. zur Konjugatfreisetzung) oder als Reaktionsschicht (z.B. zur Derivatisierung, zur Immobilisierung von Biokomponenten oder für den Ablauf chemischer und/oder immunchemischer Reaktionen) verwendet werden.

Geeignete Materialien für die verschiedenen funktionellen Schichten sind:
für die Probenvorbereitung - z.B. Membrane aus fibrösem Material zur Seperation von Plasma und roten Blutkörperchen, die beispielsweise von der Firma Pall Biosupport als "Hemadyne-Membran" erhältlich sind. Es können aber auch Filterpapiere aus Cellulose bzw. regenerierter Cellulose für diese Funktion eingesetzt werden.

Reagenzträgerschicht - hierfür kann ebenfalls Papier aus 100 % Cellulose oder aktiviertes Nylon 66, wobei die Oberflächen aktiviert oder modifiziert sein können, um die Fließeigenschaften zu verändern (kommerziell von der Firma Pall Biosupport unter der Handelsbezeichnung "Prodyne oder ACCUWIK-Membrane" erhältlich) oder speziell für laterale Durchflußsysteme Polyesterträger mit modifizierter Oberfläche, bei denen die Fließeigenschaften steuerbar sind, verwendet werden.

Reaktionsschichten - als sogenannte Nitroflowmembrane aus Nitrocellulose, PVDF (Polyvinyldifluorid)-Membran (kommerziell erhältlich bei Firma Millipor mit Handelsnamen "Immobilon"), wobei auch hier die Oberflächen modifiziert sein können.

Allgemein können fibröse Materialien, Cellulose, Nitrocellulose, Polypropylen, Polycarbonat, Polyvinyldifluorid, Hydrogele (z.B. Dextran, Acrylamid, Agar-Agar, Carrageenan, Alginsäure), Polyelektrolyten (z.B. Acrylsäure, Poly-L-Lysin, Poly-L-Glutaminsäure) oder Kernspur- oder Glasfasermembranen verwendet werden.

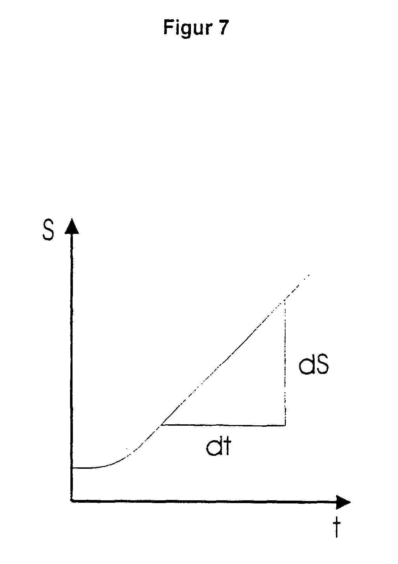

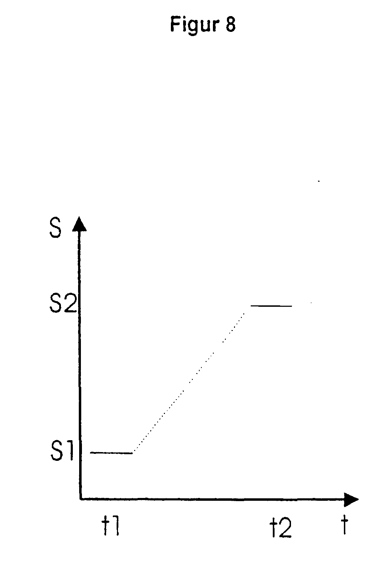

In den Figuren 7 und 8 sind prinzipielle Möglichkeiten für die Auswertung der Meßsignale dargestellt.

In der Figur 7 ist die Intensität des gemessenen Fluoreszenzsignales zeitabhängig dargestellt. Mit dem linearen Anstieg der Intensität des Fluoreszenzsignales genügt es, den Signalanstieg durch Differenzierung zu bestimmen, da der Anstieg mit der zeitlichen Änderung der Fluophormenge, die mit der erfindungsgemäßen Vorrichtung gemessen werden kann, korreliert. Dadurch kann die Meßzeit sehr kurz gehalten werden, da der Anstieg der Fluoreszenzintensität nur über einen kurzen Zeitraum bestimmt werden muß, unabhängig, ob dies zu Beginn oder einem späteren Zeitpunkt, bei der Durchführung des chemischen bzw. biochemischen Assay's erfolgt. Es ist lediglich der Sättigungsbereich zu beachten und zu berücksichtigen, daß nur in einem Zeitbereich gemessen wird, in dem eine zeitliche Änderung des Fluoreszenzintensitätssignals erfaßbar ist.

Abweichend hiervon ist in der Figur 8 eine andere Möglichkeit prinzipiell dargestellt. Hierbei wird die Differenz eines Anfangs- und Endwertes gebildet und zur Auswertung herangezogen. Dabei wird zunächst ein Grundsignal S₁ vor der Zugabe des zu bestimmenden Analyten zur Zeit t₁ aufgenommen und im Anschluß an die Analytzugabe zu einem vorgebbaren Zeitpunkt t₂ ein Endwert S₂ der gemessen Fluoreszenzintensität bestimmt. Die Analytkonzentration kann dann durch die Differenz der Werte S₂ und S₁ bestimmt werden. Die Differenz zwischen den Zeiten t₂ und t₁ muß dabei jedoch so groß sein, daß sich ein Gleichgewicht ausgebildet hat.

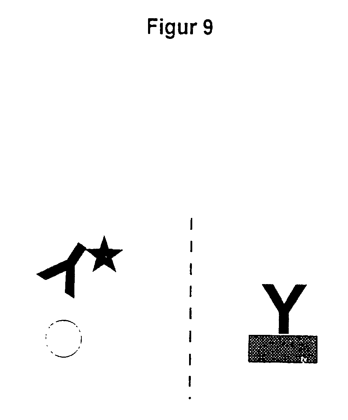

In den Figuren 9 bis 16 sind mögliche, mit der Erfindung durchführbare Assayformate dargestellt.

Die Figur 9 zeigt dabei ein Sandwich-Assayformat, das praktisch nur für hochmolekulare Verbindungen (Proteine, u.a.) geeignet ist.

Dieses Sandwich-Format kann dabei prinzipiell in einer Vorrichtung, wie sie in der Figur 5 oder Figur 6 dargestellt ist, durchgeführt werden, wobei mindestens eine funktionelle Schicht zu verwenden ist.

Dabei soll der Analyt mit dem markierten Antikörper erst inkubiert und anschließend zur Evanescentfeldanregung und entsprechender Fluoreszenz in den Detektionsbereich der Bodenplatte 3 geleitet werden.

Eine andere Möglichkeit zur Durchführung eines Sandwich-Assayformates in sequentieller Form, wird so durchgeführt, daß zuerst der Analyt und dann der markierte Antikörper schrittweise den Sandwich ausbilden.

Weitere Möglichkeiten zur Immobilisierung des Antikörpers im Bodenplattenbereich sind:
1. Adsorption
2. kovalente Bindung
3. Affinitätsbindung (z.B. A-Protein A/G oder nach Biotinylierung an Avidin)
4. durch Hybridisierung eines sich am Antikörper befindlichen Nukleinsäuremarkers (einzelsträngige RNA oder DNA) an eine immobilisierte einzelsträngige Nukleinsäure (RNA oder DNA) mit komplementärer Sequenz.

Die Beschichtung des Bodenplattenbereiches zur Evanescentfeldanregung mit Protein A/G, Avidin, u.a. bietet außerdem die Möglichkeit, ein universelles Element (für verschiedenste Analyte) herzustellen.

Eine besonders vorteilhafte Ausführung sieht die Vorinkubation des Analyten mit einem biotinylierten (Fänger-)Antikörper und einem fluoreszenzmarkierten (Detektor-)Antikörper vor. Die beiden Antikörper können beispielsweise simultan oder sequentiell aus funktionalisierten Schichten freigesetzt werden. Der gesamte Immunkomplex wird dann durch Anbindung an eine mit Avidin (alternativ Streptavidin oder Neutravidin)- beschichtete Sensoroberfläche gebunden. Ausschlaggebend für die Signalbildung ist die sehr hohe Affinität zwischen Biotin und Avidin; dies führt zu einer Verbesserung der Sensitivität des Assays.

Bei dieser Ausführung kann auch eine Vorrichtung gemäß Figur 2 in Verbindung mit zwei verschiedenen Markierungsstoffen verwendet werden und so die Konzentrationsbestimmung für dann zwei verschiedene Analyte quasi-gleichzeitig auch unabhängig von den jeweiligen Anbindungsorten im Aufnahmebereich durchgeführt werden, so daß die Anbindung der markierten Biokomponenten nicht örtlich selektiv erfolgen muß.

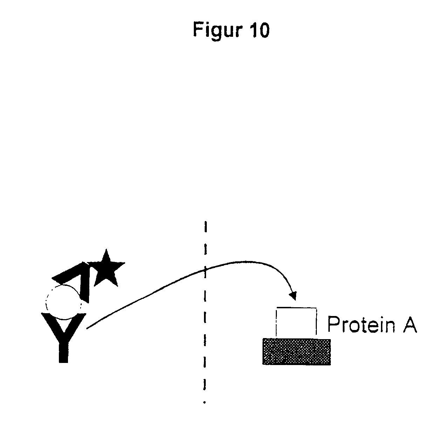

Es kann aber auch ein Assayformat durchgeführt werden, bei dem ein Antikörper und ein markiertes Antikörperfragment (z.B. ein Fc-Teil oder ein ScFv-Fragment) simmultan mit dem Analyt inkubiert werden, wie dies in Figur 10 gezeigt ist. Dort bindet nur der komplette Antikörper (an Protein A/G oder nach Biotinylierung auch an Avidin), dadurch entfällt die Erfordernis einer Inkubation. Bei diesem Format besteht die prinzipielle Möglichkeit, den verwendeten Aufbau zu regenerieren. Dies ist aber bei Avidin/Biotin nicht möglich.

Bei der simmultanen Inkubation von Analyt, Antikörper und markiertem Antikörperfragment bei einem Sandwich-Assay, wie es in Figur 10 dargestellt ist, kann das markierte Antikörperfragment und der Antikörper beispielsweise in der Funktionsschicht 27, des in Figur 5 dargestellten Beispieles, enthalten sein.

Anstelle der Immobilisierung eines Fängerantikörpers können auch andere, den Analyt bindende, Biokomponenten immobilisiert werden (z.B. Protein A/G im Fall eines Sandwich-Assay's zur Bestimmung von Antikörpern).

Bei allen Sandwich-Assayformaten tritt ein direkt proportionaler Zusammenhang zwischen der Konzentration des Analyten und dem Signal auf.

Eine oder mehrere Komponenten der immunchemischen Reaktion können außerdem auf funktionellen Schichten bereitgestellt werden, wie dies beispielsweise für eine Konjugatfreisetzung der Fall ist.

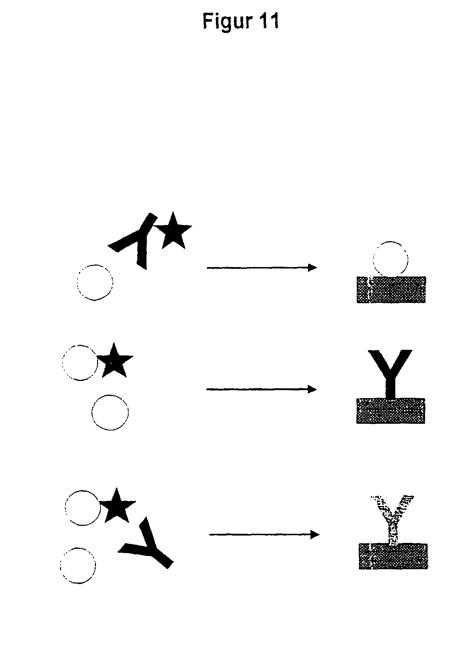

In der Figur 11 sind Möglichkeiten für Titrations-/Kompetitions-Formate dargestellt, die sich untereinander durch eine sequentielle oder gleichzeitige Inkubation der Immunkomponenten unterscheiden. Diese beiden Assayformate eignen sich insbesondere für die Bestimmung niedermolekularer Verbindungen, die keinen Sandwich ausbilden können.

Außerdem haben die in Figur 11 gezeigten Assayformate keinen direkt proportionalen Zusammenhang zwischen der Analytkonzentration und der Intensität des gemessenen Fluoreszenzsignales. Es besteht also ein umgekehrt proportionaler Zusammenhang.

So können bei dem oberen in der Figur 11 gezeigten Beispiel der markierte Antikörper beispielsweise in der funktionellen Schicht 27, beim in Figur 5 gezeigten Beispiel, vorhanden sein.

Das mittlere Beispiel der Figur 11 kann so ausgebildet sein, daß ein markierter Analyt, z.B. ebenfalls in dieser Schicht enthalten sein kann. Die untere Darstellung der Figur 11 kann so durchgeführt werden, daß ein markierter Analyt beispielsweise in der funktionellen Schicht 26 und ein Antikörper in Schicht 27 des in Figur 5 dargestellten Beispieles enthalten sind. Die Durchführung des unteren Beispiels, das in Figur 11 gezeigt ist, kann aber auch so durchgeführt werden, daß ein Antikörper in der funktionellen Schicht 26 und der markierte Analyt in der Schicht 27, bei dem in der Figur 5 dargestellten Beispiel, enthalten sind.

Daraus folgt, daß bei den in der Figur 11 gezeigten Assayformaten entweder der Analyt oder der Antikörper immobilisiert werden können (vgl. oberes und mittleres Beispiel der Figur 11). Somit können auch die bei den Sandwich-Assayformaten beschriebenen Methoden zumindest teilweise eingesetzt werden. So kann ein generischer anti-Antikörper (vgl. unteres Beispiel in Figur 11) oder aber auch Protein A/G (nach Biotinylierung des spezifischen Antikörpers auch Avidin) immobilisiert sein. In diesem Fall, dient die immobilisierte Biokomponente ausschließlich zur Anreicherung des zugesetzten spezifischen Antikörpers und kann daher im Überschuß immobilisiert werden.

Nachfolgend sollen weitere Assayformate mit direkt proportionalem Zusammenhang zwischen Analytkonzentration und Fluoreszenzsignalintensität beschrieben werden.

Hierfür sind prinzipiell zwei Möglichkeiten vorhanden, wobei der jeweilige Assay mit einer zusätzlichen festen Phase oder in Lösung durchführbar ist.

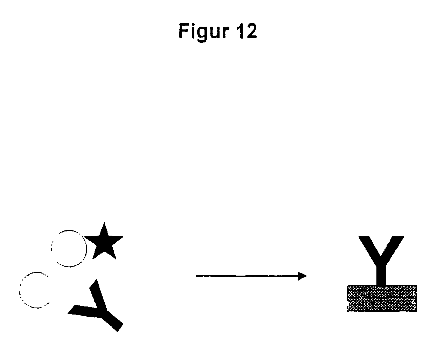

Es können beispielsweise alle Komponenten in Lösung inkubiert werden. Das in Figur 12 gezeigte Assayformat sieht eine Vorinkubation der Reaktanden und die Ausbildung eines Bindungsgleichgewichtes vor. Ein freier Analyt konkurriert mit dem markierten Analyt um die Bindung zum Antikörper, wobei auf der Bodenplatte 3 im Detektionsbereich der gleiche Antikörper immobilisiert ist, der auch in der Lösung enthalten ist.

Die Immobilisierung kann, wie bei Sandwich-Assayformaten durchgeführt werden.

Da nur freier, d.h. nicht-Antikörper-gebundener markierter Analyt bestimmt wird, resultiert ein direkt proportionaler Zusammenhang zwischen der Analytkonzentration und dem Fluoreszenzsignal.

Außerdem dient die immobilisierte Biokomponente ausschließlich der Anreicherung des Hapten-Fluorophor-Konjugates und kann somit im Überschuß immobilisiert sein. Durch Immobilisierung eines spezifischen Antikörpers kann ein entsprechender Aufbau der erfindungsgemäßen Vorrichtung jedoch nur für jeweils einen Analyten eingesetzt werden.

Das in Figur 12 gezeigte Assayformat kann mit einer Vorrichtung, wie sie in Figur 5 dargestellt ist, durchgeführt werden, wenn in der funktionellen Schicht 26 ein markierter Analyt und in der funktionellen Schicht 27 Antikörper enthalten sind.

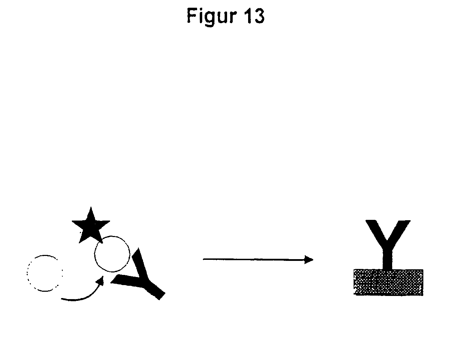

Für den Fall, daß anstelle des markierten Analyten, ein markiertes Analyt Analogon verwendet wird, das eine deutlich herabgesetzte Affinität zum Antikörper aufweist, kann auch ein bereits beschriebener Verdrängungsassay durchgeführt werden. Dies ist in der Figur 13 dargestellt. Wobei ein markierter Analyt oder ein Analyt Analogon beispielsweise in der funktionellen Schicht 28, des in Figur 6 gezeigten Beispieles, enthalten sein kann.

Es kann aber auch eine zusätzliche feste Phase ausgenutzt werden, die entweder in einem separaten Reaktionsraum oder als funktionelle Schicht direkt auf dem Detektionsbereich der Bodenplatte 3 untergebracht sein kann.

Die zusätzliche feste Phase kann prinzipiell die gleichen Funktionen ausüben, wie die funktionellen Schichten.

Die Verwendung eines separaten Reaktionsraumes (z.B. ein Inkubationsröhrchen), wie dies beispielsweise der Probencontainer 10, der in den Figuren 3 und 4 gezeigt ist, sein kann, hat den Vorteil, daß generische, d.h. für alle Analyten verwendbare Aufbauten eingesetzt werden können.

Auf diesen universellen Aufbau wird nicht ein spezifischer, sondern ein generischer anti-Antikörper oder aber Protein A/G, Avidin (nach Biotinylierung des Antikörpers) u.a. immobilisiert. Da lediglich eine Biokomponente oberhalb der Bodenplatte 3, angereichert wird, können die immobilisierten Komponenten im Überschuß aufgebracht werden.

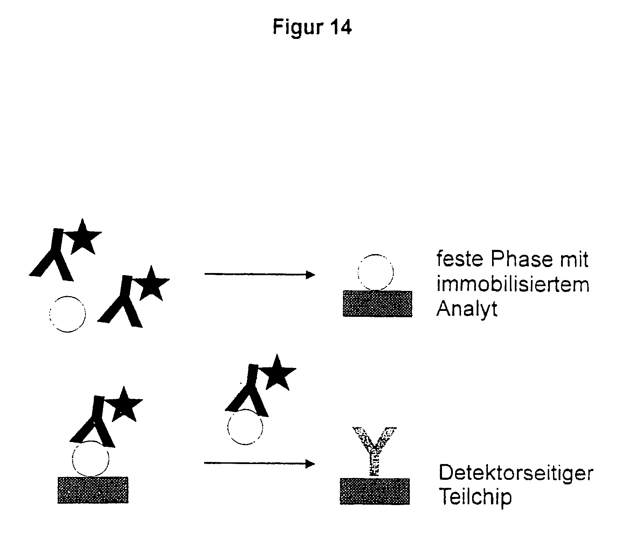

Dabei kann generell so verfahren werden, daß eine der Immunkomponenten (der markierte Antikörper oder markierte Analyt) auf einer festen Phase zurückgehalten wird, beispielsweise einer funktionellen Schicht mit Hapten-Protein-Konjugat. Dabei wird nur bei Anwesenheit von freien Analyten ein Teil der markierten Komponenten nicht an die feste Phase gebunden und kann anschließend oberhalb der Bodenplatte 3 im Detektionsbereich gemessen werden. Dieser Sachverhalt ist im in der Figur 14 gezeigten Beispiel schematisch dargestellt.

Dabei konkurriert freier und auf der festen Phase immobilisierter Analyt um die Bindung zum spezifischen Antikörper, wie dies in einem ersten Schritt oben in Figur 14 dargestellt ist.

Die feste Phase wird nur von Antikörpern, die zuvor an Analyt gebunden haben, passiert, wie dies im unteren Teil der Figur 14 dargestellt ist. Demzufolge kann nur Analyt gebundener Antikörper, beispielsweise von einem generischen anti-Antikörper detektiert werden. Auch hier besteht ein direkt proportionaler Zusammenhang zwischen der Analytkonzentration und der Intensität der gemessenen Fluoreszenz.

Wird im konkreten Fall der Figur 14 eine Membran als zusätzliche feste Phase verwendet, die in dem Aufbau integriert wird, so kann beispielsweise die funktionelle Schicht 26 (Reservoir für den markierten Antikörper) und die feste Phase als Schicht 27, des in der Figur 5 gezeigten Beispieles, bei dem in der Figur 14 gezeigtem Beispiel, verwendet werden.

Als feste Phasen können verschiedene Materialien dienen, von den sich insbesondere Membranen leicht als funktionelle Schichten integrieren lassen. Solche Membranen können Nitrocellulose, Immuno-Dyne, Konjugat-Release-Membranen, regenerierte Cellulose, u.a. sein. Dabei kann die jeweilige Biokomponente durch Adsorption, kovalente Bindung oder durch Affinitätsbindung immobilisiert werden. Haptene können als Hapten-Protein-Konjugat immobilisiert werden.

Gegenüber transversal durchflossenen Membranen bieten lateral durchflossene Membranen und gepackte Säulen Vorteile durch eine wiederholte Gleichgewichtseinstellung und ermöglichen eine quantitative Anbindung der Biokomponenten. Geeignete Materialien für gepackte Säulen sind:
Sepharose, Porosmedien u.a.

Die Wandung eines möglichen verwendbaren Gefäßes, die beispielsweise die Wandung des genannten Probencontainers 10 bzw. die Zuleitungen, können ebenfalls als feste Phase dienen und beispielsweise Polysterolgefäβe oder Glaskapillaren sein. Weiter können Partikelsuspensionen, in denen die Probe eine Suspension mit festen Partikeln (Magnetpartikel, Latex, u.a.) sein kann. Diese Partikel können dann durch Anlegen eines Magnetfeldes oder durch Filtration im Nachgang getrennt werden.

Mit der Erfindung ist es auch möglich, sogenannte Verdrängungs-Assayformate durchzuführen, wobei hierfür prinzipiell zwei Varianten möglich sind. Dabei kann die Verdrängung auf einer zusätzlichen festen Phase in einer funktionellen Schicht oder extern, also nicht in einer integrierten funktionellen Schicht oder direkt auf der Bodenplatte 3 im Detektionsbereich stattfinden.

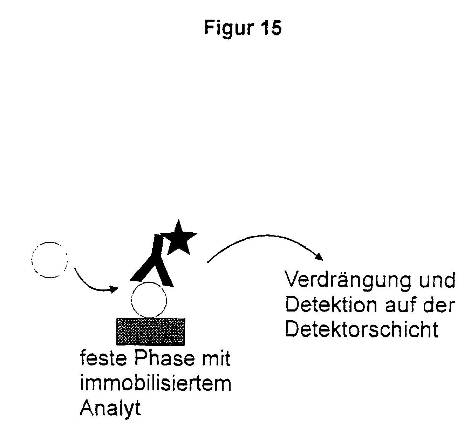

In der Figur 15 ist ein Beispiel eines Verdrängungsassay's mit zusätzlicher fester Phase prinzipiell dargestellt. Die feste Phase kann entweder der genannte Probencontainer 10, eine Zuleitung oder eine funktionelle Schicht sein. Dabei wird ein markierter Antikörper oder Analyt durch spezifische Ligand/Rezeptor-Wirkung gebunden. Durch Zugabe eines freien Analyten kann die Verdrängung der Biokomponente erreicht werden.

Die feste Phase kann beispielsweise die funktionelle Schicht 26, im Beispiel nach Figur 5, sein.

Im in der Figur 15 gezeigten Beispiel wird der markierte Antikörper auf der Bodenplatte 3 im Detektionsbereich von einem generischen anti-Antikörper oder aber Protein A/G, Avidin u.a. gebunden.

Wird aber gegenteilig verfahren und ein markierter Analyt an einen immobilisierten Antikörper gebunden und anschließend verdrängt, so wird im Detektionsbereich auf der Bodenplatte 3 ein spezifischer, gegen den Analyten gerichteter Antikörper immobilisiert. Da in jedem Fall immer die verdrängte Komponente detektiert wird, besteht ein direkter proportionaler Zusammenhang zwischen der Konzentration des jeweiligen Analyten und der Fluoreszenzsignalintensität.

Die Verdrängung kann aber auch direkt im Detektionsbereich auf der Bodenplatte 3 als sehr einfache Assaykonfiguration durchgeführt werden, da lediglich eine Probe durch das Element geführt wird.

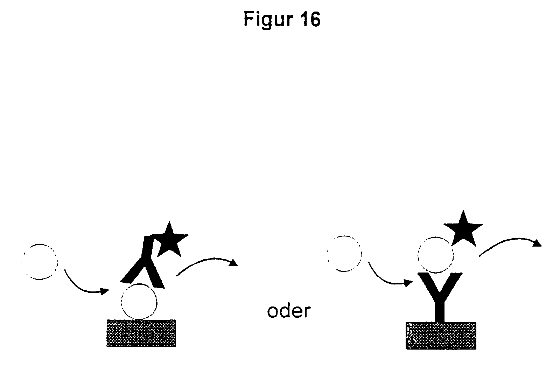

Es finden keine Vorinkubationen oder ähnliche Schritte statt. Eine Konditionierung der Probe kann durch Integration entsprechender funktioneller Schichten erzielt werden. Auch hier bieten sich zwei verschiedene Möglichkeiten an, den Analyten oder den spezifischen Antikörper zu immobilisieren, wie dies in Figur 16 gezeigt ist.

Dabei wird jeweils die Abnahme des Fluoreszenzintensitätssignals gemessen, so daß ein umgekehrt proportionaler Zusammenhang zwischen der Analytkonzentration und der Fluoreszenzsignalintensität auftritt. Der Absolutwert des Anstieges des Fluoreszenzintensitätssignals ist jedoch der Analytkonzentration direkt proportional und kann in der bereits beschriebenen Form ausgewertet werden.

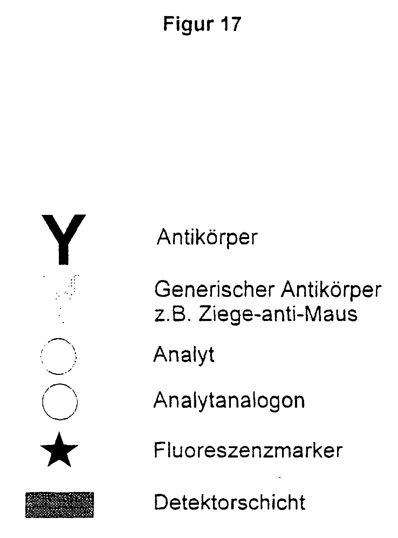

Die Figur 17 dient als generelle Legende für die in den Figuren 9 bis 16 gezeigten verschiedenen Assay-Formate.

## Patentansprüche

1. Vorrichtung zur Durchführung von Fluoreszenzimmunotests mittels Evanescentfeldanregung, bei der mindestens eine nahezu monochromatisches Licht aussendende Lichtquelle (7, 7'), Lichtstrahlen mit einer Fluoreszenz eines an einem chemischen oder biochemischen Partner eines allgemeinen Rezeptor-Ligand Systems gebundenen Markierungsstoffes hervorrufenden Wellenlänge, in einem durch eine vorgebbare Eindringtiefe d für das evanescente Feld bestimmenden Winkel α auf eine Grenzfläche zwischen einer optisch transparenten Bodenplatte (1) aus Material dessen Brechungsindex n₁ größer ist als der Brechungsindex n₂ des Materials oberhalb der Grenzfläche und einem küvettenförmig ausgebildeten Aufnahmebereich (2) für die Probe richtend, angeordnet ist,
der Aufnahmebereich (2) auf der entgegengesetzt zur Bodenplatte (1) angeordneten Seite mit einer Deckplatte (3) abgedeckt ist, wobei zwischen Bödenplatte (1) und Deckplatte (3) oder einem Zuflußbereich für die Probe zum Aufnahmebereich (2) mindestens eine durch Saug-, Druck- oder Kapillarkraft lateral oder transversal durchflossene funktionelle Schicht (26, 27, 28, 28', 29) zur Probenvorbereitung angeordnet ist und
ein Detektor (5) zur Erfassung des Fluoreszenzlichtes gleichzeitig zur Bodenplatte (1), wie die Lichtquelle, angeordnet ist.

2. Vorrichtung nach Anspruch 1,
**dadurch gekennzeichnet, daß** zwischen Bodenplatte (1) und Deckplatte (3) ein den küvettenförmigen Aufnahmebereich (2) ausbildender Abstandshalter (4) angeordnet ist.

3. Vorrichtung nach Anspruch 1 oder 2,
**dadurch gekennzeichnet, daß** in der Deckplatte (3) eine Zufluß- und eine Abflußöffnung (9, 11) vorhanden sind.

4. Vorrichtung nach einem der Ansprüche 1 bis 3,
**dadurch gekennzeichnet, daß** die funktionnelle(n) Schicht(en) (25, 27, 28, 28', 29) aus fibrösem Material, Cellulose, Nitrocellulose, Polypropylen, Polycarbonat, Polyvinyldifluorid bestehen oder aus einem Hydrogel oder aus Polyelektrolyten oder aus Kernspur- oder Glasfasermembranen bestehen oder als gepackte Säule ausgebildet sind.

5. Vorrichtung nach einem der Ansprüche 1 bis 4,
**dadurch gekennzeichnet, daß** mindestens eine funktionelle Schicht (29) im Aufnahmebereich (2) im direkten Kontakt mit der Bodenplatte (3) steht.

6. Vorrichtung nach einem der Ansprüche 1 bis 5,
**dadurch gekennzeichnet, daß** mehrere durch Trennschichten (25, 25') getrennte funktionelle Schichten (26, 27) eine Verbindung der Zufluß- und Abflußöffnung (9, 11) über den Aufnahmebereich (2) mittels Durchbrechungen ermöglichend, alternierend übereinander angeordnet sind.

7. Vorrichtung nach Anspruch 5,
**dadurch gekennzeichnet, daß** mindestens zwei unterschiedlich funktionelle Schichten in einer Ebene nebeneinander angeordnet sind.

8. Vorrichtung nach einem der Ansprüche 1 bis 5,
**dadurch gekennzeichnet, daß** mehrere funktionelle Schichten (28, 28', 29) unmittelbar übereinander angeordnet sind.

9. Vorrichtung nach einem der Ansprüche 1 bis 8,
**dadurch gekennzeichnet, daß** ein ein definiertes Probenvolumen vorgebender Probencontainer (10) so angeordnet ist, daß eine Verbindung zwischen küvettenförmigen Aufnahmebereich (2) und Probencontainer (10) gebildet ist.

10. Vorrichtung nach einem der Ansprüche 1 bis 9,
**dadurch gekennzeichnet, daß** eine feste Phase im Probencontainer (10) in einem Zuflußkanal, im Aufnahmebereich (2) oder eine funktionelle Schicht als solche ausgebildet ist.

11. Verfahren zur Durchführung von Fluoreszenzimmunotests mittels Evanescentfeldanregung, bei dem ein Probenvolumen mittels Saug-, Druckwirkung oder Kapillarkräften zur Vorbereitung der Probe durch eine oder mehrere funktionelle Schicht(en) (26, 27, 28, 28', 29) und danach durch den küvettenförmigen Aufnahmebereich (2) geführt, die je nach biochemischen Assay zu bestimmenden markierten chemischen oder biochemischen Komponenten an der Oberfläche im Aufnahmebereich (2) gebunden und durch Evanescentfeldanregung mit einem Detektor (5) das Fluoreszenzlicht zeitabhängig gemessen wird.

12. Verfahren nach Anspruch 11,
**dadurch gekennzeichnet, daß** mit der/den funktionellen Schicht(en) (26, 27, 28, 28', 29) eine Filterung, Separation, eine Eleminierung von interferierenden Substanzen, die Freisetzung von Reagenzien oder chemische Reaktionen durchgeführt wird/werden.

13. Verfahren nach Anspruch 11 oder 12,
**dadurch gekennzeichnet, daß** der Anstieg der gemessenen Fluoreszenzlichtintensität zur Bestimmung der Analytkonzentration benutzt wird.

14. Verfahren nach Anspruch 11 oder 12,
**dadurch gekennzeichnet, daß** die Differenz zweier in vorgebbaren Abständen gemessenen Fluoreszenzintensitätssignalen (S₁ und S₂) zur Bestimmung der Analytkonzentration benutzt wird.

15. Verfahren nach einem der Ansprüche von 11 bis 14, **dadurch gekennzeichnet, daß** biochemische Assays von allgemeinen Rezeptor-Ligand Systemen, wie Antikörper-Antigen, Lectin-Kohlenhydrat, DNA oder RNA-komplenentäre Nukleinsäure, DNA oder RNA-Protein, Hormonrezeptor, Enzym-Enzymcofaktoren, Protein G oder Protein A-Immunglobin oder Avidin-Biotin durchgeführt werden.

16. Verfahren nach einem der Ansprüche von 11 bis 15, **dadurch gekennzeichnet, daß** Sandwich-, Titrations-, Kompetitions und/oder Verdrängungsassays durchgeführt werden.

## Claims

1. Device for carrying out fluorescence immunoassays by means of evanescent field excitation, in which at least one light source (7, 7') which emits almost monochromatic light and directs light beams of a wavelength which causes fluorescence of a labelling substance bound to a chemical or biochemical partner of a general receptor/ligand system at an angle α to be defined by a predeterminable penetration depth d for the evanescent field onto an interface between an optically transparent base plate (1) made of a material whose refractive index n₁ is greater than the refractive index n₂ of the material above the interface and a reception region (2) in the form of a cuvette for the sample is arranged,
the reception region (2) is covered on the opposite side from the base plate (1) with a cover plate (3), at least one functional layer (26, 27, 28, 28', 29) for sample preparation, through which flow takes place laterally or transversely due to suction, pressure or capillary force, being arranged between the base plate (1) and the cover plate (3) or a region for sample inflow to the reception region (2), and
a detector (5) for picking up the fluorescent light is arranged, like the light source, at the same time as the base plate (1).

2. Device according to Claim 1, **characterized in that** between the base plate (1) and the cover plate (3) a spacer (4) which forms the reception region (2) in the form of a cuvette is arranged.

3. Device according to Claim 1 or 2, **characterized in that** the cover plate (3) contains an inflow orifice and a discharge orifice (9, 11).

4. Device according to any of Claims 1 to 3, **characterized in that** the functional layer(s) (26, 27, 28, 28', 29) are made of fibrous material, cellulose, nitrocellulose, polypropylene, polycarbonate, polyvinyl difluoride or are made of a hydrogel or of polyelectrolytes or of nuclear track or glass fibre membranes or are designed as a packed column.

5. Device according to any of Claims 1 to 4, **characterized in that** at least one functional layer (29) in the reception region (2) is in direct contact with the base plate (3).

6. Device according to any of Claims 1 to 5, **characterized in that** a plurality of functional layers (26, 27) separated by separating layers (25, 25') and making possible a connection of the inflow orifice and discharge orifice (9, 11) via the reception region (2) by means of openings are arranged alternately above one another.

7. Device according to Claim 5, **characterized in that** at least two layers with different functions are arranged side-by-side in a plane.

8. Device according to any of Claims 1 to 5, **characterized in that** a plurality of functional layers (28, 28', 29) are arranged immediately above one another.

9. Device according to any of Claims 1 to 8, **characterized in that** a sample container (10) predetermining a defined sample volume is arranged such that a connection between the reception region (2) in the form of a cuvette and the sample container (10) is formed.

10. Device according to any of Claims 1 to 9, **characterized in that** a solid phase is formed in the sample container (10), in an inflow channel, in the reception region (2), or a functional layer is formed as such a phase.

11. Method for carrying out fluorescent immunoassays by means of evanescent field excitation, in which a sample volume is passed through one or more functional layer(s) (26, 27, 28, 28', 29) by means of suction, pressure or capillary forces in order to prepare the sample and is then passed through the reception region (2) in the form of a cuvette, the labelled chemical or biochemical components, to be determined depending on the biochemical assay, are bound to the surface in the reception region (2) and the fluorescent light is measured as a function of time by evanescent field excitation using a detector (5).

12. Method according to Claim 11, **characterized in that** the functional layer(s) (26, 27, 28, 28', 29) is/are used to carry out a filtering, separation, an elimination of interfering substances, the release of reagents or chemical reactions.

13. Method according to Claim 11 or 12, **characterized in that** the increase in the fluorescent light intensity measured is used for determining the analyte concentration.

14. Method according to Claim 11 or 12, **characterized in that** the difference between two fluorescent intensity signals (S₁ and S₂) measured in predeterminable intervals is used for determining the analyte concentration.

15. Method according to any of Claims 11 to 14, **characterized in that** biochemical assays of general receptor-ligand systems such as antibody-antigen, lectin-carbohydrate, DNA or RNA-complementary nucleic acid, DNA or RNA-protein, hormone receptor, enzyme-enzyme cofactors, protein G or protein A-immunoglobin or avidine-biotin are carried out.

16. Method according to any of Claims 11 to 15, **characterized in that** sandwich, titration, competition and/or displacement assays are carried out.

## Revendications

1. Dispositif pour effectuer des analyses immunologiques par fluorescence en excitant un champ évanescent, dans lequel est disposée au moins une source lumineuse (7, 7') émettant une lumière quasiment monochromatique, orientant des rayons lumineux présentant une longueur d'onde entraînant la fluorescence d'un marqueur lié à un agent chimique ou biochimique d'un complexe récepteur-ligand général, selon un angle α déterminé en fonction d'une profondeur de pénétration d pouvant être prédéfinie pour le champ évanescent, sur une surface limite entre une plaque inférieure (1) transparente d'un point de vue optique, réalisée dans un matériau dont l'indice de réfraction n₁ est supérieur à l'indice de réfraction n₂ du matériau se trouvant au-dessus de la surface limite, et une zone de réception conçue en forme de cuvette (2), destinée à l'échantillon,
la zone de réception (2), du côté opposé à la plaque inférieure (1), est revêtue d'une plaque de recouvrement (3), moyennant quoi, entre la plaque inférieure (1) et la plaque de recouvrement (3) ou une zone d'admission permettant à l'échantillon d'atteindre la zone de réception (2), est disposée au moins une couche fonctionnelle (26, 27, 28, 28') faisant l'objet d'un passage latéral ou transversal sous l'effet d'une force d'aspiration, de pression, ou capillaire, pour préparer les échantillons, et
un détecteur (5) destiné à enregistrer la lumière fluorescente est disposé en même temps que la source lumineuse par rapport à la plaque inférieure (1).

2. Dispositif selon la revendication 1, **caractérisé en ce que**, entre la plaque inférieure (1) et la plaque de recouvrement (3) est disposé un séparateur (4) donnant à la zone de réception une forme de cuvette (2).

3. Dispositif selon la revendication 1 ou 2, **caractérisé en ce que**, dans la plaque de recouvrement (3), il existe un orifice d'admission et un orifice d'évacuation (9, 11).

4. Dispositif selon l'une quelconque des revendications 1 à 3, **caractérisé en ce que** la ou les couche(s) fonctionnelle(s) (26, 27, 28, 28', 29) est (sont) constituée(s) de matières fibreuses, cellulose, nitrocellulose, polypropylène, polycarbonate, difluorure de polyvinyle ou d'un hydrogel ou de polyélectrolytes ou de membranes de traces nucléaires ou de fibres de verre, ou sont conçues sous la forme d'une colonne compacte.

5. Dispositif selon l'une quelconque des revendications 1 à 4, **caractérisé en ce qu'**au moins une couche fonctionnelle (29) dans la zone de réception (2) est en contact direct avec la plaque inférieure (1).

6. Dispositif selon l'une quelconque des revendications 1 à 5, **caractérisé en ce que** plusieurs couches fonctionnelles (26, 27) séparées par des couches de séparation (25, 25') sont disposées alternativement les unes au-dessus des autres en permettant de relier l'orifice d'admission et l'orifice d'évacuation (9, 11) par l'intermédiaire de la zone de réception (2) à l'aide de perforations.

7. Dispositif selon la revendication 5, **caractérisé en ce qu'**au moins deux couches ayant des fonctions différentes sont disposées à un même niveau en étant adjacentes l'une à l'autre.

8. Dispositif selon l'une quelconque des revendications 1 à 5, **caractérisé en ce que** plusieurs couches fonctionnelles (28, 28', 29) sont disposées directement les unes au-dessus des autres.

9. Dispositif selon l'une quelconque des revendications 1 à 8, **caractérisé en ce qu'**un conteneur d'échantillons (10) déterminant un volume d'échantillons défini est disposé de telle sorte qu'il se forme une liaison entre la zone de réception en forme de cuvette (2) et le conteneur d'échantillons (10).

10. Dispositif selon l'une quelconque des revendications 1 à 9, **caractérisé en ce qu'**une phase solide est formée dans le conteneur d'échantillons (10), dans un canal d'admission, dans la zone de réception (2) ou une couche fonctionnelle en tant que telle est formée.

11. Procédé pour effectuer des analyses immunologiques par fluorescence en excitant un champ évanescent, dans lequel un volume d'échantillons est acheminé par l'effet d'aspiration, de pression ou des forces capillaires pour préparer l'échantillon, au travers d'une ou plusieurs couches fonctionnelles (26, 27, 28, 28', 29) puis au travers de la zone de réception en forme de cuvette (2), qui est lié à des composants chimiques ou biochimiques marqués à déterminer en fonction du dosage biochimique, au niveau de la surface dans la zone de réception (2), et la lumière fluorescente est mesurée par rapport au temps à l'aide d'un détecteur (5) en excitant le champ évanescent.

12. Procédé selon la revendication 11, **caractérisé en ce que**, à l'aide de la ou des couche(s) fonctionnelle(s) (26, 27, 28, 28', 29), on réalise un filtrage, une séparation, une élimination des substances interférentes, la libération des réactifs ou des réactions chimiques.

13. Procédé selon la revendication 11 ou 12, **caractérisé en ce que** l'augmentation de l'intensité de la lumière fluorescente mesurée est utilisée pour mesurer la concentration en analytes.

14. Procédé selon la revendication 11 ou 12, **caractérisé en ce que** la différence entre deux signaux d'intensité de fluorescence (S₁ et S₂) mesurés à des distances pouvant être prédéfinies est utilisée pour déterminer la concentration en analytes.

15. Procédé selon l'une quelconque des revendications 11 à 14, **caractérisé en ce qu'**on réalise des dosages biochimiques de complexes récepteur-ligand généraux, comme les complexes anticorps-antigène, lectine-hydrate de carbone, acide nucléique complémentaire d'ADN ou d'ARN, protéines d'ADN ou d'ARN, récepteur hormonal, cofacteurs enzyme-enzyme, protéine G ou protéine A-immunoglobine, ou avidine-biotine.

16. Procédé selon l'une quelconque des revendications 11 à 15, **caractérisé en ce qu'**on réalise des dosages « sandwich », de titrage, des dosages avec compétition et/ou déplacement.
